Europäisches Patentamt

European Patent Office

Office européen des brevets

(19)

(11) Veröffentlichungsnummer: **0 010 177**
**B1**

(12) # EUROPÄISCHE PATENTSCHRIFT

(45) Veröffentlichungstag der Patentschrift:
**21.08.85**

(51) Int. Cl.⁴: **A 61 F 2/64**

(21) Anmeldenummer: **79103542.1**

(22) Anmeldetag: **20.09.79**

(54) **Künstliches Kniegelenk.**

(30) Priorität: **27.09.78 DE 2841999**

(43) Veröffentlichungstag der Anmeldung:
**30.04.80 Patentblatt 80/9**

(45) Bekanntmachung des Hinweises auf die Patenterteilung:
**21.08.85 Patentblatt 85/34**

(84) Benannte Vertragsstaaten:
**AT BE CH FR GB IT LU NL SE**

(56) Entgegenhaltungen:
**DE - A - 2 432 766**
**DE - B - 2 332 993**
**DE - C - 808 743**
**DE - U - 1 793 820**
**DE - U - 1 825 882**
**FR - A - 1 039 077**
**GB - A - 1 091 015**
**GB - A - 1 303 738**

(73) Patentinhaber: **Otto Bock Orthopädische Industrie Besitz- und Verwaltungs-Kommanditgesellschaft, Industriestrasse, D-3428 Duderstadt (DE)**

(72) Erfinder: **Glabiszewski, Richard, Im Puttfeld 1, D-3428 Duderstadt 13 (DE)**
Erfinder: **Näder, Max, Hindenburgring 39, D-3428 Duderstadt (DE)**

(74) Vertreter: **Gramm, Werner, Dipl.-Ing. et al, Patentanwälte Gramm + Lins Theodor-Heuss-Strasse 2, D-3300 Braunschweig (DE)**

## Beschreibung

Die Erfindung betrifft ein künstliches Kniegelenk, mit einer Aufnahme für einen praktisch vollständig erhaltenen Oberschenkel (Exartikulationsamputation), unterhalb derer für das Gelenkoberteil und ein darunter angeordnete Gelenkunterteil jeweils ein vorderes und ein hinteres Drehgelenk vorgesehen sind, von denen jeweils die beiden vorderen und die beiden hinteren durch Lenker verbunden sind.

Derartige Kniegelenke gehören zu den Vier-Achs-Gelenken, die an sich schon seit mehreren Jahrzehnten bekannt sind. Die neueren Gelenke dieser Art sind auch bei Exartikulationsamputationen, d. h. Amputationen, bei denen der Oberschenkel praktisch vollständig erhalten bleibt und die Trennung durch Abnahme des kompletten Unterschenkels erfolgt, brauchbar. Ein derartiges Kniegelenk ist aus der DE-B-2 332 993 bekannt. Die Verbindung zwischen dem Gelenkoberteil und dem Gelenkunterteil wird durch zwei Lenkerpaare bewirkt, die jeweils an ihren beiden Enden durch Achsen miteinander verbunden sind. Die Lage der Drehgelenke ist durch komplizierte Winkelbeziehungen festgelegt, die genauestens einzuhalten sind, damit eine gute Funktion des Gelenkes gewährleistet ist. Die beiden vorderen Lenker, die die beiden vorderen Drehgelenke miteinander verbinden, sind im Vergleich zu den hinteren Lenkern sehr kurz ausgebildet und weisen eine wesentlich stärkere Krümmung auf. Die beiden vorderen Drehgelenke sind daher relativ nahe zueinander gelegen, während der Abstand zwischen den hinteren Drehgelenken etwa doppelt so groß ist.

Die Funktion dieses bekannten Kniegelenks ist an sich nicht zu beanstanden, da es eine ausreichende Standstabilität des ausgestreckten Beines und eine gute Drehpunktlage innerhalb des Knies beim Beugen gewährleistet. Die Bewegung des natürlichen Knies kann mit diesem Gelenk relativ gut nachgeahmt werden.

Als Nachteil des bekannten Gelenks ist anzusehen, daß eine Vielzahl von kompliziert geformten Teilen zum Aufbau des Kniegelenks erforderlich ist. Demzufolge gestaltet sich auch die Montage des Kniegelenks aufwendig. Ein weiterer Nachteil ist darin zu sehen, daß für das Kniegelenk ein relativ großer Raumbedarf erforderlich ist, der dazu führt, daß der üblicherweise verwendete kosmetische Überzug relativ dünn ausgeführt sein muß und daher kein einwandfreies Aussehen mehr erhält und darüber hinaus schneller durchscheuert.

Aus der französischen Patentschrift 1 039 077 ist ein künstliches Vier-Achs-Gelenk bekannt, das aus zwei vorderen und einem hinteren Lenker besteht. Der hintere Lenker ist kürzer ausgebildet als die vorderen und gegenüber den vorderen zum Gelenkoberteil hin etwas geneigt. Die Befestigung der Lenker geschieht sowohl im Gelenkunterteil als auch im Gelenkoberteil innerhalb des Oberschenkels bzw. des Unterschenkels, so daß dieses Gelenk nicht für eine Exartikulationsamputation geeignet ist, da es voraussetzt, daß ein wesentlicher Teil des Oberschenkels durch eine Prothese ersetzt ist, in die die oberen Enden der Lenker hineinragen können. Die Funktionsweise dieses Gelenks beruht entscheidend darauf, daß sich die oberen Drehpunkte bereits innerhalb des Oberschenkels befinden, während bei einem für Exartikulationsamputationen geeigneten künstlichen Kniegelenk die Drehpunkte erst unterhalb des kompletten Oberschenkels sitzen dürfen, woraus sich völlig andere Bewegungsverhältnisse ergeben.

Der Erfindung liegt die Aufgabe zugrunde, ein künstliches Kniegelenk der eingangs erwähnten Art zu erstellen, dessen Aufbau weniger kompliziert ist und das einen geringeren Raumbedarf hat.

Diese Aufgabe wird erfindungsgemäß dadurch gelöst, daß die Verbindungslinie zwischen den beiden Drehgelenken des Gelenkunterteils bei vertikal stehendem Unterschenkel etwa horizontal verläuft, daß die Abstände zwischen den Drehgelenken des Gelenkoberteils und den zugehörigen Drehgelenken des Gelenkunterteils gleich sind, daß sich die Abstände zwischen den beiden Drehgelenken des Gelenkoberteils, zwischen den beiden Drehgelenken des Gelenkunterteils und zwischen den Drehgelenken des Gelenkoberteils und den zugehörigen Drehgelenken des Gelenkunterteils etwa wie 1 : 2 : 4 verhalten und daß die Verbindungslinien zwischen den vorderen Drehgelenken und den hinteren Drehgelenken, wenn das Gelenk in der Lage ist, die einem gestreckten Bein entspricht, zum Gelenkoberteil hin zueinander geneigt sind.

Das erfindungsgemäße Kniegelenk ist gegenüber den bekannten Kniegelenken, die für Exartikulationsamputationen geeignet sind, wesentlich einfacher aufgebaut. Die für das erfindungsgemäße Kniegelenk erforderlichen Teile können sehr einfach ausgestaltet sein und lassen sich daher einfach herstellen. Das gesamte Kniegelenk benötigt in allen drei Richtungen weniger Platz als die bisherigen Kniegelenke. Darüber hinaus läßt sich das erfindungsgemäße Kniegelenk einfach montieren und unterliegt nicht den kritischen Winkelbeziehungen der bekannten Kniegelenke. Durch die erfindungsgemäße Anordnung der Drehgelenke können diese sehr nahe beinander positioniert werden, so daß lediglich geringe Hebelkräfte auftreten, die von den Lenkern aufgenommen werden müssen. Daher ist es auch möglich, das erfindungsgemäße Kniegelenk mit nur zwei Lenkern aufzubauen.

Die Herstellungsvorteile lassen sich dadurch nicht vergrößern, daß die Lenker eine gleiche Form aufweisen. Sie können dann vorzugsweise aus gleichen Teilen gebildet sein, die beispielsweise um 180° zuzüglich bzw. abzüglich eines spitzen Winkels gegeneinander verdreht im Kniegelenk eingesetzt sind. Die Lenker haben dann vorzugsweise die Form eines Kreisbogenabschnitts.

Während bei den bekannten Kniegelenken immer unterschiedliche Lenker, insbesondere bezüglich ihrer Länge, vorn und hinten eingesetzt worden sind und dies für unbedingt notwendig gehalten wurde, ermöglicht das erfindungsgemäße Kniegelenk die Verwendung gleichlanger oder völlig gleicher Lenker vorn und hinten. Ohne irgendwelche Abstriche in der Funktion des Kniegelenks machen zu müssen, kann daher die Herstellung des Kniegelenkes noch weiter vereinfacht werden. Als besonders vorteilhaft hat es sich herausgestellt, wenn die Verbindungslinien zwischen den beiden Drehgelenken des Gelenkunterteils einerseits und einem Drehgelenk des Gelenkoberteils und dem zugehörigen Drehgelenk des Gelenkunterteils andererseits etwa senkrecht aufeinander stehen.

Eine weitere wesentliche Vereinfachung der Montage des Kniegelenks läßt sich dadurch erzielen, daß die Lenker mit Einsteckzapfen in Passung in entsprechende Bohrungen in den Gelenkteilen eingesetzt sind. Dadurch entfällt die Notwendigkeit, für jedes Drehgelenk eine eigene Schraube vorzusehen.

Eine besonders stabile Ausführung wird dann erreicht, wenn die Lenker aus einem Winkelprofil gebildet sind.

Die Einsteckzapfen können vorzugsweise als Hohlzapfen ausgeführt sein, in die die Zapfen eines Verriegelungselements einführbar sind. Auf diese Weise läßt sich eine äußerst simple Verriegelung der gesamten Knieanordnung mit Hilfe einer einzigen Schraube erzielen, wenn in die beiden in das Gelenkunterteil eingeführten Einsteckzapfen die Zapfen des Verriegelungselementes eingeführt sind und einer der beiden Zapfen durch das Drehgelenk hindurchragend in das Verriegelungselement eingeschraubt ist. Die gesamte Anordnung kann daher mit der in das Verriegelungselement eingeschraubten Schraube zusammengehalten werden, wobei das Verriegelungselement die beiden unteren Einsteckzapfen am Herausgleiten aus der entsprechenden Bohrung hindert und durch die wechselseitige Anbringung der beiden Lenker die oberen Einsteckzapfen automatisch von den unteren mit gehalten werden, ohne daß für sie eine eigene zusätzliche Befestigung erforderlich wäre.

In einer bevorzugten Ausführungsform ist das Verriegelungselement drehfest mit dem mit der Schraube versehenen Einsteckzapfen verbunden. Dabei kann das Verriegelungselement auf seinem mit der Schraube versehenen Ende vorzugsweise mit einer Reibungsbremse versehen sein. Auf diese Weise läßt sich die Funktion der einen Schraube, die die gesamte Anordnung hält, noch dahingehend erweitern, daß mit ihr die Gangregulierung ausgeführt wird. Je nach der Einstellung der Schraube wird das Knie eine mehr oder weniger gebremste Pendelbewegung ausführen.

In einer besonders einfachen Ausführungsform ist die Reibungsbremse durch einen Stapel kreisförmiger Scheiben gebildet, durch den die Schraube hindurchragt. Die Scheiben können bei abwechseln aus Metall und aus Kunststoff gebildet sein.

Die erfindungsgemäße Ausgestaltung des Kniegelenks, insbesondere die Verwendung von nur zwei Lenkern ermöglicht die platzsparende Unterbringung eines für die Gangregulierung erforderlichen Federelements. Dieses ist vorzugsweise zwischen dem hinteren Lenker und dem Gelenkunterteil eingespannt. Die Vorspannung des Federelements ist dabei durch einen auf ein Gewinde aufgeschraubten Anschlagbolzen für die Spiralfeder verstellbar.

Das Gelenkunterteil kann mit einem Anschlag versehen sein, der die Bewegung des vorderen Lenkers nach hinten begrenzt. Der Anschlag ist vorzugsweise verstellbar, in einer einfachen Ausführungsform dadurch, daß er einen Gewindeansatz aufweist, mit dem er in ein Gewindeloch des Gelenkunterteils eingeschraubt ist.

Das komplette Kniegelenk einschließlich aller für die Gangregulierung notwendigen Teile kann daher auf einem minimalen Raum untergebracht werden, da alle für die Gangregulierung vorgesehenen Teile in dem Raum zwischen den Lenkern angeordnet sein können. Hier liegt ein erheblicher Vorteil gegenüber allen bekannten Kniegelenken der in Rede stehenden Art.

Die Erfindung soll im folgenden anhand eines in der Zeichnung dargestellten Ausführungsbeispiels näher erläutert werden. Es zeigt

Fig. 1 die Seitenansicht eines erfindungsgemäßen Kniegelenks,

Fig. 1a einen Schnitt entlang der Linie I-I,

Fig. 2 das Kniegelenk gemäß Fig. 1 von vorn,

Fig. 3 die Stellung der Lenker beim gebeugten Kniegelenk.

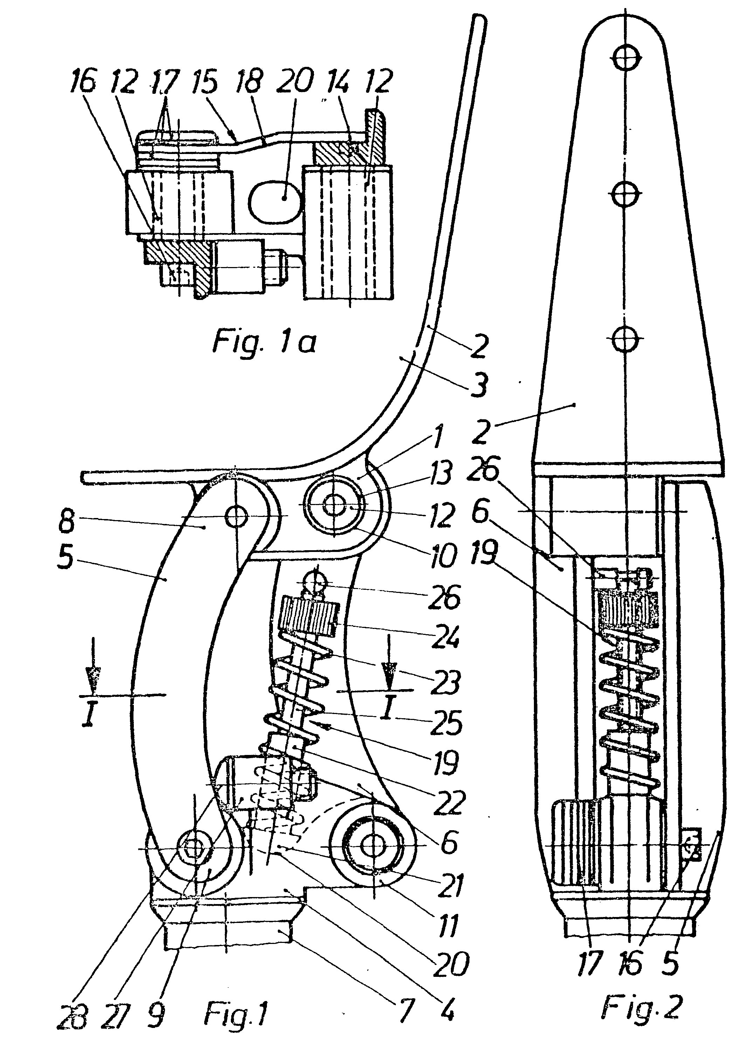

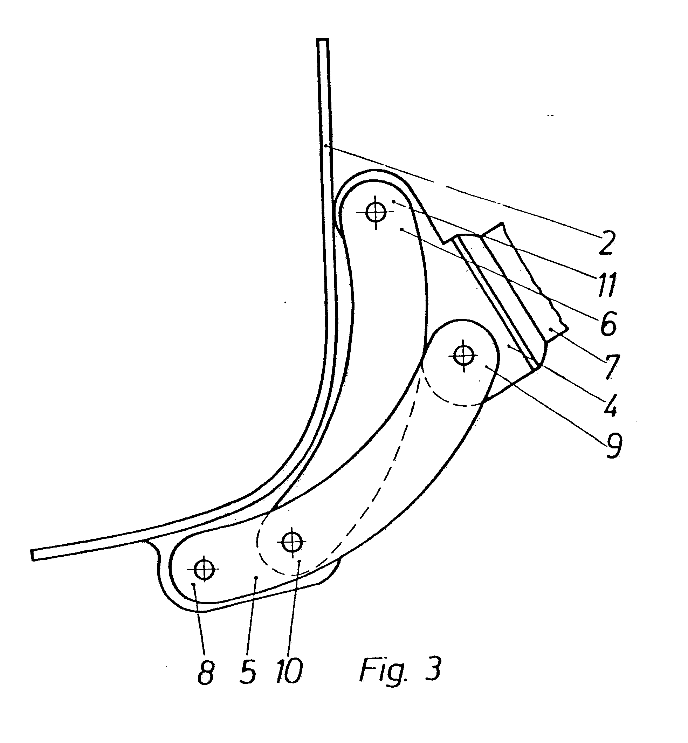

Das in den Fig. 1 bis 3 dargestellte Kniegelenk besteht aus einem Gelenkoberteil 1, das einstückig mit einem etwa L-förmig ausgebildeten Befestigungsanker 2 für einen Stumpfschaft 3 verbunden ist. Das Gelenkoberteil 1 ist mit einem Gelenkunterteil 4 über zwei Lenker 5, 6 verbunden. An dem Gelenkunterteil 4 wird das Unterschenkelrohr 7 einer Unterschenkelprothese befestigt.

Der in Gehrichtung gesehen vordere Lenker 5 ist an dem Gelenkoberteil 1 mit einem Gelenk 8 und an dem Gelenkunterteil 4 mit einem Drehgelenk 9 verbunden. Hinter diesen beiden Drehgelenken 8, 9 befinden sich zwei weitere Drehgelenke 10, 11, die durch den hinteren Lenker 6 verbunden sind.

Die Lenker 5, 6 weisen beide die gleiche Form auf und stellen einen nach hinten gekrümmten Kreisbogenabschnitt dar. Sie sind aus einem Winkelprofil gebildet, das ihnen eine besondere Steifheit verleiht. Von vorn gesehen befinden sich die beiden Lenker auf verschiedenen Seiten der Kniegelenkmitte. Die Drehgelenke 8, 9, 10, 11 werden durch an den Lenkern 5, 6 befindliche Einsteckzapfen 12 gebildet, die mit Passung in entsprechende Bohrungen 13 in den Gelenkteilen 1, 4 eingesetzt sind.

Die Drehgelenke 9, 11 in dem Gelenkunterteil 4 liegen horizontal nebeneinander, betrachtet bei einem senkrecht stehenden Unterschenkelrohr 7.

Die Verbindungslinie zwischen den beiden Drehgelenken 9, 11 steht in etwa senkrecht auf der Verbindungslinie zwischen den beiden hinteren Drehgelenken 10, 11. Die Verbindungslinie zwischen den beiden vorderen Drehgelenken 8, 9 bildet mit der Vertikalen einen kleinen Winkel, ist also gegen die Verbindungslinie zwischen den beiden hinteren Drehgelenken 10, 11 um diesen Winkel geneigt. Daraus ergibt sich, daß der Abstand zwischen den beiden Drehgelenken 8, 10 im Gelenkoberteil 1 geringer ist, als der Abstand zwischen den beiden Drehgelenken 9, 11 im Gelenkunterteil 4. In dem dargestellten Ausführungsbeispiel beträgt der Abstand zwischen den oberen Drehgelenken 8, 10 ca. 2 cm, der Abstand zwischen den beiden unteren Drehgelenken 9, 11 ca. 4 cm und der Abstand zwischen den beiden hinteren Drehgelenken 10, 11 ca. 8 cm. Bei dieser Anordnung liegt der scheinbare Drehpunkt des Kniegelenks anatomisch richtig innerhalb des Knies. Auch bei der Beugung des Kniegelenkes verändert er seine Lage nicht wesentlich. Das Kniegelenk bewirkt bei der Beugung eine anatomisch richtige Verkürzung des Beines, so daß eine genügende Freiheit für die Fußprothese vorhanden ist und der Prothesenträger ungehindert gehen kann.

Die Einsteckzapfen 12 sind als Hohlzapfen ausgeführt, in die Zapfen 14 eines Verriegelungselements 15 einsteckbar sind. Das Verriegelungselement 15 greift in die beiden Einsteckzapfen 12 im Gelenkunterteil 4 ein, und zwar mit einem kurzen Zapfen 14 in dem hinteren Drehgelenk 11 und in dem vorderen Drehgelenk 9 mit einem als Schraube 16 ausgebildeten Zapfen, der durch den Lenker 5 und den Einsteckzapfen 12 hindurchragend in das Verriegelungselement 15 eingeschraubt ist. Diese Schraube stellt das einzige Befestigungsmittel für alle Drehgelenke 8, 9, 10, 11 dar. Zugleich wird die Schraube 16 noch zur Verstellung der Achsfriktion ausgenutzt, indem das Verriegelungselement 15 als Reibungsbremse ausgebildet ist. Es besteht nämlich an seinem die Schraube 16 aufnehmenden Ende aus einem Stapel von Kreisscheiben 17, die abwechselnd aus Metall und aus Kunststoff gebildet sind. Die Metallscheiben, mit Ausnehme derjenigen Metallscheibe, die mit der Federlasche 18 des Verriegelungselements 15, an der sich der kurze Zapfen 14 befindet, drehfest miteinander verbunden, während die Kunststoffscheiben frei drehbar sind. Die drehfeste Verbindung der Metallscheiben untereinander ist dadurch bewirkt, daß die äußerste Metallscheibe einen rechteckigen Ansatz aufweist, der in eine entsprechende Ausnehmung in dem Drehgelenk 9 hineinpaßt. Eine entsprechende rechteckige Ausnehmung weisen die übrigen Metallscheiben auf. In dem rechteckigen Ansatz befindet sich auch das Gewinde für die Aufnahme der Schraube 16. Auf diese Weise wirkt jede Metallscheibe mit jeder Kunststoffscheibe als Reibfläche bei einer Drehung des Drehgelenks 9. Je nach der Stärke des Drucks durch die Schraube 16 ist die Reibung mehr oder weniger stark, so daß durch die Einstellung der Schraube 16 die Pendelbewegung des Unterschenkels reguliert werden kann.

Zur Rückstellung des nach hinten abgeknickten Unterschenkels wird bei Kniegelenken ein Federelement 19 als sogenannte Vorbringerfeder verwendet. Diese ist in dem dargestellten Ausführungsbeispiel zwischen dem hinteren Lenker 6 und dem Gelenkunterteil 4 eingespannt. Das Gelenkunterteil 4 weist hierzu eine Mulde 20 zwischen den Drehgelenken 9, 11 auf, in die ein entsprechend geformter Kunststoffknopf 21 am Ende eines Kunststoffzylinders 22 hineinpaßt. Der Kunststoffknopf dient als Anschlag für eine Spiralfeder 23, die auf ihrer anderen Seite durch einen Anschlagbolzen 24 gehalten wird, der auf einer Gewindestange 25 in seiner Position veränderbar ist. Die Gewindestange 25 ist in dem Kunststoffzylinder 22 verschiebbar gelagert. In eine vorgesehene Bohrung des hinteren Lenkers 6 ist ein Bolzen 26 eingesteckt, der mit einer breiten Nut als Widerlager für das Federelement 19 dient. Das Ende der Gewindestange 25 ist dazu zylindrisch ausgehöhlt, so daß es teilweise den Bolzen 26 umfaßt. Das Federelement wird beim Beugen des Knies zusammengedrückt. Dadurch übt es eine verstärkte Rückstellkraft aus, die den Unterschenkel beim Gehen nach vorne drückt. Durch die Anordnung der Drehpunkte ist gewährleistet, daß beim vollständigen Einknicken des Knies die Federkraft nicht mehr in Richtung Kniestreckung wirkt, sondern das Einknicken des Knies noch unterstützt. Auf diese Weise kann das eingeknickte Knie in einer Ruhestellung gehalten werden. Die Rückstellkraft der Vorbringerfeder ist mit Hilfe des verstellbaren Anschlagbolzens 24 auf die individuellen Gegebenheiten des Prothesenträgers einstellbar.

Wie in Fig. 1 zu erkennen ist, weist das Gelenkunterteil 4 eine hochgezogene Lasche 27 auf, in der sich ein Gewindeloch befindet. In das Gewindeloch ist ein Anschlag 28 mit einem Gewindeansatz eingeschraubt. Der Anschlag 28 ist nach vorn ausgerichtet, so daß er die Bewegung des vorderen Lenkers 5 nach hinten begrenzt. Durch die Ausbildung des Anschlags 28 mit dem Gewindeansatz kann auch seine Lage verändert werden, so daß auch die Ruhestellung des Kniegelenks beim gestreckten Bein in der Weise einstellbar ist, daß die Neigung des Befestigungsankers 2 und damit des Oberschenkelstumpfes beim gestreckten Kniegelenk von der Einstellung des Anschlags 28 abhängt.

Somit sind alle für die Funktion des Kniegelenks wesentlichen Teile in dem Raum zwischen Gelenkoberteil 1 und Gelenkunterteil 4 in vertikaler Richtung und zwischen den beiden Lenkern 5, 6 in seitlicher Richtung untergebracht. Das erfindungsgemäße Kniegelenk besteht aus sehr wenigen, einfach geformten Einzelteilen, die eine leichte Montage erlauben. Aufgrund der kompakten Ausführung kann ein kosmetischer Überzug in der notwendigen Dicke aufgebracht werden, der ein einwandfreies ästhetisches Aussehen ermöglicht.

**Patentansprüche**

1. Künstliches Kniegelenk mit einer Aufnahme (2) für einen praktisch vollständig erhaltenen Oberschenkel (Exartikulationsamputation), unterhalb derer für das Gelenkoberteil (1) und ein darunter angeordnetes Gelenkunterteil (4) jeweils ein vorderes (8, 9) und ein hinteres (10, 11) Drehgelenk vorgesehen sind, von denen jeweils die beiden vorderen (8, 9) und die beiden hinteren (10, 11) durch Lenker (5, 6) verbunden sind, dadurch gekennzeichnet, daß die Verbindungslinie zwischen den beiden Drehgelenken (9, 11) des Gelenkunterteils (4) bei vertikal stehendem Unterschenkel etwa horizontal verläuft, daß die Abstände zwischen den Drehgelenken (8, 10) des Gelenkoberteils (1) und den zugehörigen Drehgelenken (9, 11) des Gelenkunterteils (4) gleich sind, daß sich die Abstände zwischen den beiden Drehgelenken (8, 10) des Gelenkoberteils (1), zwischen den beiden Drehgelenken (9, 11) des Gelenkunterteils (4) und zwischen den Drehgelenken des Gelenkoberteils und den zugehörigen Drehgelenken des Gelenkunterteils 1 : 2 : 4 verhalten und daß die Verbindungslinien zwischen den vorderen Drehgelenken (8, 9) und den hinteren Drehgelenken (10, 11), wenn das Gelenk in der Lage ist, die einem gestreckten Bein entspricht, zum Gelenkoberteil (1) hin zueinander geneigt sind.

2. Kniegelenk nach Anspruch 1, dadurch gekennzeichnet, daß die Lenker (5, 6) eine gleiche Form aufweisen.

3. Kniegelenk nach Anspruch 2, dadurch gekennzeichnet, daß die Lenker (5, 6) aus gleichen Teilen gebildet sind, die um 180° zuzüglich bzw. abzüglich eines kleinen Winkels gegeneinander verdreht im Kniegelenk eingesetzt sind.

4. Kniegelenk nach einem der Ansprüche 1 bis 3, dadurch gekennzeichnet, daß die Verbindungslinie zwischen den beiden Drehgelenken (9, 11) des Gelenkunterteils (4) einerseits und einem Drehgelenk (8) bzw. (10) des Gelenkoberteils und dem zugehörigen Drehgelenk (9) bzw. (11) des Gelenkunterteils (4) andererseits etwa senkrecht aufeinanderstehen.

5. Kniegelenk nach einem der Ansprüche 1 bis 4, dadurch gekennzeichnet, daß die Lenker (5, 6) die Form eines Kreisbogenabschnitts aufweisen.

6. Kniegelenk nach einem der Ansprüche 1 bis 5, dadurch gekennzeichnet, daß die Lenker (5, 6) aus einem Winkelprofil gebildet sind.

7. Kniegelenk nach einem der Ansprüche 1 bis 6, dadurch gekennzeichnet, daß zwischen dem hinteren Lenker (6) und dem Gelenkunterteil (4) ein Federelement (19) eingespannt ist.

8. Kniegelenk nach Anspruch 7, dadurch gekennzeichnet, daß die Vorspannung des Federelements (19) durch einen auf ein Gewinde (25) aufgeschraubten Anschlagbolzen (24) für die Spiralfeder (23) verstellbar ist.

9. Kniegelenk nach einem der Ansprüche 1 bis 8, dadurch gekennzeichnet, daß das Gelenkunterteil (4) einen Anschlag (28) aufweist, der die Bewegung des vorderen Lenkers (5) nach hinten begrenzt.

10. Kniegelenk nach Anspruch 9, dadurch gekennzeichnet, daß der Anschlag (28) verstellbar ist.

11. Kniegelenk nach Anspruch 10, dadurch gekennzeichnet, daß der Anschlag (28) einen Gewindeansatz aufweist, mit dem er in ein Gewindeloch des Gelenkunterteils (4) eingeschraubt ist.

12. Kniegelenk nach einem der Ansprüche 1 bis 11, dadurch gekennzeichnet, daß die Lenker (5, 6) mit Einsteckzapfen (12) in Passung in entsprechende Bohrungen (13) in den Gelenkteilen (1, 4) eingesetzt sind.

13. Kniegelenk nach Anspruch 12, dadurch gekennzeichnet, daß die Einsteckzapfen (12) als Hohlzapfen ausgeführt sind, in die Zapfen (14, 16) eines Verriegelungselements (15) einführbar sind.

14. Kniegelenk nach Anspruch 13, dadurch gekennzeichnet, daß das Kniegelenk mittels in die beiden in das Gelenkunterteil (4) eingeführten Einsteckzapfen (12) eingeführte Zapfen (14, 16) des Verriegelungselements (15) gehalten wird, von denen einer (16) durch das Drehgelenk (9) hindurchragend in das Verriegelungselement (15) geschraubt ist.

15. Kniegelenk nach Anspruch 14, dadurch gekennzeichnet, daß das Verriegelungselement (15) drehfest mit dem mit der Schraube (16) versehenen Einsteckzapfen (12) verbunden ist.

16. Kniegelenk nach Anspruch 15, dadurch gekennzeichnet, daß das Verriegelungselement (15) auf seinem mit der Schraube (16) versehenen Ende mit einer Reibungsbremse (17) versehen ist.

17. Kniegelenk nach Anspruch 16, dadurch gekennzeichnet, daß die Reibungsbremse durch einen Stapel kreisförmiger Scheiben (17) gebildet ist, durch die die Schraube (16) hindurchragt und von denen im wesentlichen jede zweite drehfest mit dem Einsteckzapfen (12) verbunden ist.

18. Kniegelenk nach Anspruch 17, dadurch gekennzeichnet, daß die Scheiben (17) abwechselnd aus Metall und aus Kunststoff gebildet sind.

**Claims**

1. An artificial knee joint, comprising a receptacle (2) for a virtually complete upper thigh (exarticulate amputation), wherein below said receptacle (2) an anterior (8, 9) and a posterior (10, 11) pivoting joint are provided each for an upper (1) and a lower joint part (4) positioned below said upper joint part, the two respective anterior (8, 9) and posterior (10, 11) pivoting joints being linked by connecting bars (5, 6), characterized in that the connecting line extending between the two pivoting joints (9, 11) of said lower joint part (4) is substantially horizontal when the lower thigh is in a vertical position, the distances between the pivoting joints (8, 10) of said upper joint part (1) and the respective pivoting joints (9, 11) of said lower joint part (4) are euqual, the distances between the two pivoting joints (8, 10) of said upper joint part (1), between the two pivoting joints (9, 11) of said lower joint part (4) and between the pivoting joints of the upper joint part and the respective pivoting joints of the lower joint part have a ratio of approximately 1 : 2 : 4, the connecting lines extending between the anterior pivoting joints (8, 9) and the posterior pivoting joints (10, 11) are inclined towards each other in the direction of the upper joint part (1), when the joint is in a position corresponding to an extended leg.

2. The joint according to claim 1, characterized in that said connecting bars (5, 6) are of identical shape.

3. The joint according to claim 2, characterized in that said connecting bars (5, 6) consists of identical parts being inserted into the knee joint substantially at an angle of 180° relative to each other, with addition or substraction, respectively, of a small angle.

4. The joint according to one of the claims 1 to 3, characterized in that the connecting lines extending between the two pivoting joints (9, 11) of said lower joint part (4) on the one hand and a pivoting joint (8) or (10), respectively, of said upper joint part and the corresponding pivoting joint (9) or (11), respectively, of the lower joint part (4) on the other hand, are substantially perpendicular to each other.

5. The joint according to one of the claims 1 to 4, characterized in that said connecting bars (5, 6) each have the shape of a circular arc segment.

6. The joint according to one of the claims 1 to 5, characterized in that said connecting bars (5, 6) consist of angle sections.

7. The joint according to one of the claims 1 to 6, characterized in that a spring element (19) is clamped between the posterior connecting bar (6) and said lower joint part (4).

8. The joint according to claim 7, characterized in that the pretensioning of said spring element (19) is adjustable by means of a stop pin (24) for the helical spring (23), said stop pin being screwed onto a thread (25).

9. The joint according to one of the claims 1 to 8, characterized in that the lower joint part (4) comprises a stop (28) delimiting the rearward movement of the anterior connecting bar (5).

10. The joint according to claim 9, characterized in that said stop (28) is adjustable.

11. The joint according to claim 10, characterized in that said stop (28) comprises a projection with an external thread, said lower joint part having a threaded bore receiving said projection.

12. The joint according to one of the claims 1 to 11, characterized in that said connecting bars (5, 6) are inserted by means of dowels (12) fitted into respective bores (13) of the joint parts (1, 4).

13. The joint according to claim 12, characterized in that said dowels (12) are constructed as hollow dowels, into which pins (14, 16) of a locking element (15) can be inserted.

14. The joint according to claim 13, characterized in that said knee joint is held by means of the pins (14, 16) of the locking element (15), said pins being inserted into the two dowels (12) of the lower joint part (4) and one of the pins (16) protruding through the pivoting joint (9) being screwed into the locking element (15).

15. The joint according to claim 14, characterized in that said locking element (15) is connected with the dowel (12) having a screw (16) and is secured on said dowel against rotation.

16. The joint of claim 15, characterized in that said locking element (15) comprises a friction brake (17) arranged at that end of the locking element bearing the screw (16).

17. The joint of claim 16, characterized in that said friction brake (17) includes a stack of circular discs, the screw (16) protruding through said discs and, essentially every second disc being connected with and secured on the dowel (12) against rotation.

18. The joint of claim 17, wherein said discs (17) alternately are of metal and plastic.

**Revendications**

1. Articulation artificielle du genou, comportant un organe d'accueil (2) pour une jambe supérieure conservée pratiquement complète (amputation par exarticulation), au dessous duquel il est prévu une articulation pivotante antérieure (8, 9) et une articulation pivotante postérieure (10, 11) pour chacune de deux parties de l'articulation, à savoir la partie supérieure de l'articulation (1) et une partie inférieure de l'articulation (4) disposée en dessous, tandis que le couple d'articulations antérieures (8, 9) et le couple d'articulation postérieure (10, 11) sont reliés chacun par une biellette (5, 6), caractérisée en ce que la ligne qui relie les deux articulations pivotantes (9, 11) de la partie inférieure de l'articulation (4) s'étend à peu près horizontalement lorsque la jambe inférieure est verticale, en ce que les distances qui séparent les articulations pivotantes (8, 10) de la partie supérieure de l'articulation (1) et les articulations pivotantes (9, 11) correspondantes de la partie inférieure de l'articulation (4) son égales, en ce que les distances entre les deux articulations pivotantes (8, 10) de la partie supérieure de l'articulation (1), entre les deux articulations pivotantes (9, 11) de la partie inférieure de l'articulation (4), et entre les articulations pivotantes de la partie supérieure de l'articulation et les articulations pivotantes correspondantes de la partie inférieure de l'articulation sont dans les rapports approximatifs de 1 : 2 : 4,
et en ce que les lignes qui relient les articulations pivotantes antérieures (8, 9) et les articulations pivotantes postérieures (10, 11), lorsque l'articulation est dans l'attitude qui correspond à une jambe tendue, sont inclinées l'une vers l'autre lorsque l'on va vers la partie supérieure de l'articulations (1).

2. Articulation du genou conforme à la revendication 1, caractérisée en ce que les biellettes (5, 6) présentent une même forme.

3. Articulation du genre conforme à la revendication 2, caractérisée en ce que les biellettes (5, 6) sont constituées des mêmes pièces, qui sont montées dans l'articulation du genou tournées l'une par rapport à l'autre de 180°, plus ou respectivement moins un petit angle.

4. Articulation du genou conforme à l'une des revendications 1 à 3, caractérisé en ce que la ligne qui relie les deux articulations pivotantes (9, 11) de la partie inférieure de l'articulation (4) d'une part, et celle qui relie une articulation pivotante (8) ou (10) de la partie supérieure de l'articulation (1) et l'articulation pivotante correspondante (9) ou (11) de la partie inférieure de l'articulation (4) d'autre part sont sensiblement perpendiculaires l'une à l'autre.

5. Articulation du genou conforme à l'une des revendications 1 à 4, caractérisée en ce que les biellettes (5, 6) ont la forme d'un arc de cercle.

6. Articulation du genou conforme à l'une des revendications 1 à 5, caractérisée en ce que les biellettes (5, 6) sont réalisées à partir d'une cornière.

7. Articulation du genou conforme à l'une des revendications 1 à 6, caractérisée en ce qu'un élément à ressort (19) est interposé entre la biellette postérieure (6) et la partie inférieure de l'articulation (4).

8. Articulation du genou conforme à la revendication 7, caractérisée en ce que la précontrainte de l'élément à ressort (19) est réglable au moyen d'un écrou de butée (24) vissé sur une vis (25) et destiné au ressort à boudin (23).

9. Articulation du genou conforme à l'une des revendications 1 à 8, caractérisée en ce que la partie inférieure de l'articulation (4) présente une butée (28) qui limite vers l'arrière le mouvement de la biellette antérieure (5).

10. Articulation du genou conforme à la revendication 9, caractérisée en ce que la butée (28) est réglable.

11. Articulation du genou conforme à la revendication 10, caractérisée en ce que la butée (28) présente un appendice fileté par lequel elle est vissée dans un trou taraudé de la partie inférieure de l'articulation (4).

12. Articulation du genou conforme à l'une des revendications 1 à 11, caractérisée en ce que les biellettes (5, 6) sont montées par des tourillons enfichables (12) ajustés dans des alésages (13) correspondants pratiqués dans les parties de l'articulation (1, 4).

13. Articulation du genou conforme à la revendication 12, caractérisée en ce que les tourillons enfichables (12) sont réalisés sous la forme de tourillons creux, dans lesquels peuvent rentrer les chevilles (14, 16) d'un élément de verrouillage (15).

14. Articulation du genou conforme à la revendication 13, caractérisée en ce que l'articulation du genou est maintenue au moyen de chevilles (14, 16) de l'élément de verrouillage (15) introduites dans les deux tourillons enfichables (12) introduits eux-mêmes dans la partie inférieure de l'articulation (4), chevilles dont l'une (16) traverse l'articulation pivotante (9) et en fait saillie pour être vissée dans l'élément de verrouillage (15).

15. Articulation du genou conforme à la revendication 14, caractérisée en ce que l'élément de verrouillage (15) est lié en rotation au tourillon enfichable (12) muni de la vis (16).

16. Articulation du genou conforme à la revendication 15, caractérisée en ce que l'élément de verrouillage (15) est muni, à son extrémité munie de la vis (16), d'un frein à friction (17).

17. Articulation du genou conforme à la revendication 16, caractérisée en ce que le frein à friction est constitué d'un empilement de disques circulaires (17) à travers lesquels la vis (16) passe et fait saillie, et dont en substance un sur deux est immobilisé en rotation relativement au tourillon enfichable (12).

18. Articulation du genou conforme à la revendication 17, caractérisée en ce que les disques (17) sont alternativement en métal et en matière synthétique.

Fig. 1a

Fig. 1

Fig. 2

Fig. 3